# EUROPÄISCHE PATENTANMELDUNG

(11) **EP 3 351 207 A1**
(43) Veröffentlichungstag der Anmeldung: **25.07.2018**
(21) Anmeldenummer: 17210783.1
(22) Anmeldetag: 28.12.2017
(51) Int. Cl.: A61F 2/00, A61M 25/04, A61M 25/10, A61M 3/02, A61M 25/00, B29C 31/00

(54) **REKTALKATHETER-KUNSTSTOFFBAUTEIL FÜR EINE REKTALKATHETER-BAUGRUPPE SOWIE VERFAHREN ZUM HERSTELLEN EINES DERARTIGEN REKTALKATHETER-KUNSTSTOFFBAUTEILS**

(30) Priorität: 24.01.2017 DE 102017201065
(71) Anmelder: Raumedic AG, 95213 Münchberg (DE)
(72) Erfinder: Gläsel, Björn, 95158 Kirchenlamitz (DE)
(74) Vertreter: Rau, Schneck & Hübner Patentanwälte Rechtsanwälte PartGmbB

(57) **Zusammenfassung**

Ein Rektalkatheter-Kunststoffbauteil (1) ist Bestandteil einer Rektalkatheter-Baugruppe. Das Kunststoffbauteil hat einen zylindrischen Grundkörper (2) mit einer äußeren Mantelwand. Ein Konnektor-Bauteilende (3) dient zum Anschluss eines Peripherie-Schlauches. Ein freies Bauteilende (4) dient zum rektalen Einführen. Mindestens ein längs des Grundkörpers verlaufendes Medienlumen (5) ist angeordnet zwischen mindestens einem Medien-Konnektorport (6) und mindestens einer Medienöffnung (7). Der Medien-Konnektorport ist am Konnektor-Bauteilende und die Medienöffnung ist im freien Bauteilende ausgeführt. Ein weiteres längs des Grundkörpers verlaufendes Ballon-Befülllumen (8) verläuft zwischen einem Befüll-Konnektorport (9) und einer Ballon-Befüllöffnung (10). Der Befüll-Konnektorport (9) ist am Konnektor-Bauteilende und die Ballon-Befüllöffnung ist in einem Ballon-Anschluss-Bereich des Kunststoffbauteils zum Anschluss einer Ballon-Manchette ausgeführt. Das Kunststoffbauteil ist einstückig ausgeführt. Es resultiert ein Rektalkatheter-Kunststoffbauteil für eine kostengünstiger gestaltete Rektalkatheter-Baugruppe.

## Beschreibung

Der Inhalt der deutschen Patentanmeldung DE 10 2017 201 065.7 wird durch Bezugnahme hierin aufgenommen.

Die Erfindung betrifft einen Rektalkatheter-Kunststoffbauteil für eine Rektalkatheter-Baugruppe. Ferner betrifft die Erfindung ein Verfahren zum Herstellen eines derartigen Rektalkatheter-Kunststoffbauteils sowie eine derartige Rektalkatheter-Baugruppe.

Rektalkatheter-Baugruppen sind vom Markt her bekannt, beispielsweise aus der DE 44 36 796 A1.

Es ist eine Aufgabe der vorliegenden Erfindung, den Aufbau einer Rektalkatheter-Baugruppe kostengünstiger zu gestalten.

Diese Aufgabe ist erfindungsgemäß gelöst durch ein Rektalkatheter-Kunststoffbauteil mit den im Anspruch 1 angegebenen Merkmalen.

Erfindungsgemäß wurde erkannt, dass ein die notwendigen Lumen aufweisender zylindrischer Grundkörper einerseits sowie ein Konnektor-Bauteilende zum Anschluss eines Peripherieschlauchs andererseits als einstückiges Kunststoffbauteil ausgeführt werden können. Dies erspart eine aufwendige mehrteilige Ausgestaltung, bei der die verschiedenen Komponenten gegeneinander abgedichtet und aufwendig montiert werden müssten. Auch Hygiene- und Sterilisationsprobleme entfallen. Der Grundkörper ist als Mehrlumenkörper gestaltet und kann genau zwei Lumen oder auch mehr als zwei Lumen aufweisen, beispielsweise drei Lumen, vier Lumen, fünf Lumen oder noch mehr Lumen.

Eine über einen Steg voneinander getrennte Lumengestaltung nach Anspruch 2 führt zu einem einfach gestalteten Querschnittsaufbau des Grundkörpers. Der Steg ist einstückig in den Grundkörper eingeformt.

Eine Lumengestaltung nach Anspruch 3 kann längs der Lumen verlaufende Kanten vermeiden. Die konvexen Lumenquerschnitte können kreisförmig rund, elliptisch oder oval geformt sein.

Eine Gestaltung des Rektalkatheter-Kunststoffbauteils als Spritzgussbauteil ermöglicht eine Massenproduktion.

Ein Ringanschlag nach Anspruch 5 ermöglicht eine Fixierung des Rektalkatheter-Kunststoffbauteils.

Ein weiteres Katheterlumen nach Anspruch 6 erweitert die Einsatzmöglichkeiten der mit einem solchen Rektalkatheter-Kunststoffbauteil ausgerüsteten Rektalkatheter-Baugruppe.

Ein Herstellungsverfahren nach Anspruch 7 geht aus von einem vergleichsweise einfach gestaltetem Vorformling, der insbesondere durch Spritzguss hergestellt sein kann. Der Vorformling kann ein bereits angeformtes Konnektor-Bauteilende aufweisen. Zum Herstellungsverfahren kann der Verfahrensschritt eines Formens des freien Bauteilendes gehören. Hierbei kann das freie Bauteilende insbesondere mit einer balligen Spitze ausgeführt werden, sodass das rektale Einführen des freien Bauteilendes vereinfacht ist.

Eine Medienöffnung kann über eine Rohröffnung des Vorformlings gebildet sein. Alternativ kann eine Medienöffnung durch Stanzen gemäß Anspruch 8 gebildet werden.

Die Vorteile einer Rektalkatheter-Baugruppe nach Anspruch 9 entsprechen denen, die vorstehend unter Bezugnahme auf das Rektalkatheter-Kunststoffbauteil bereits erläutert wurden. Die Ballon-Manschette kann mit dem Rektalkatheter-Kunststoffbauteil verklebt und/oder verschweißt sein.

Eine Rektalkatheter-Baugruppe mit einem Peripherieschlauch nach Anspruch 10 kann als Komplettprodukt vertrieben werden.

Ein separates Konnektor-Bauteil nach Anspruch 11 vereinfacht die Gestaltung eines Konnektor-Bauteilendes am Rektalkatheter-Kunststoffbauteil.

Ausführungsbeispiele der Erfindung werden nachfolgend anhand der Zeichnungen näher erläutert. In dieser zeigen:
- Fig. 1 bis 6: Axialschnitte durch ein Kunststoffbauteil während dessen Bearbeitung bei der Herstellung eines Rektalkatheter-Kunststoffbauteils;
- Fig. 7 und 8: zwei alternative Ausführungen einer Querschnittsgestaltung eines zylindrischen Grundkörpers des Kunststoffbauteils, jeweils ausgeführt mit zwei längs des Grundkörpers verlaufenden Lumen;
- Fig. 9: perspektivisch eine Rektalkatheter-Baugruppe mit einer weiteren Ausführung eines Rektalkatheter-Kunststoffbauteils, welches abgesehen von einer Gestaltung eines Konnektor-Bauteilendes der Ausführung des Rektalkatheter-Kunststoffbauteils nach Fig. 6 entspricht;
- Fig. 10: eine Seitenansicht der Rektalkatheter-Baugruppe nach Fig. 9;
- Fig. 11: eine Ansicht der Rektalkatheter-Baugruppe, gesehen aus Blickrichtung XI in Fig. 10;
- Fig. 12: einen Axialschnitt durch die Rektalkatheter-Baugruppe nach den Fig. 9 bis 11;
- Fig. 13: perspektivisch eine weitere Ausführung einer Rektalkatheter-Baugruppe, ausgeführt mit einer weiteren Ausführung eines Rektalkatheter-Kunststoffbauteils;
- Fig. 14: eine Seitenansicht der Rektalkatheter-Baugruppe nach Fig. 13;
- Fig. 15: eine Stirnansicht der Rektalkatheter-Baugruppe, gesehen aus Blickrichtung XV in Fig. 14;
- Fig. 16: einen Axialschnitt durch die Rektalkatheter-Baugruppe nach den Fig. 13 bis 15; und
- Fig. 17: perspektivisch einen Abschnitt eines Mehrlumen-Peripherieschlauchs, geeignet zum Anschluss an die Rektalkatheter-Baugruppe nach den Fig. 13 bis 16.

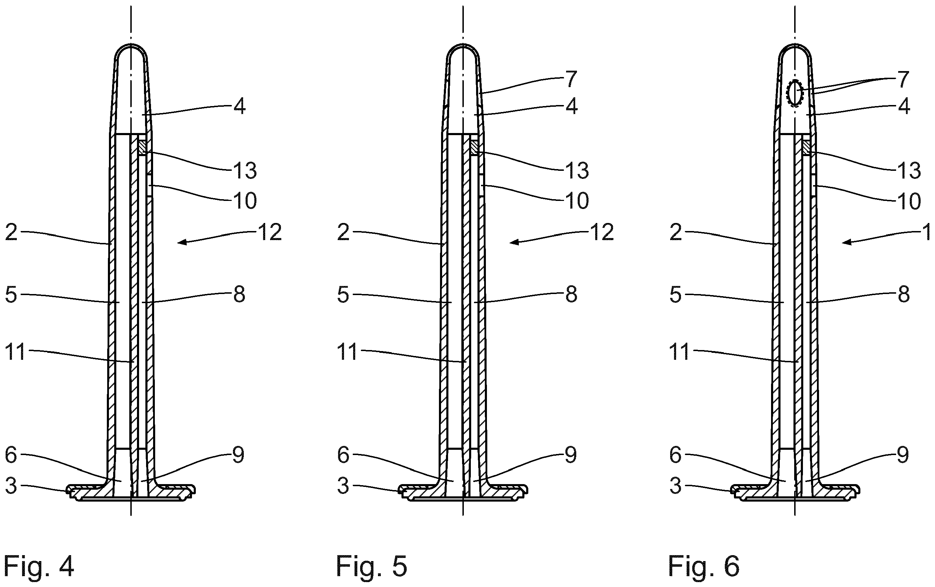

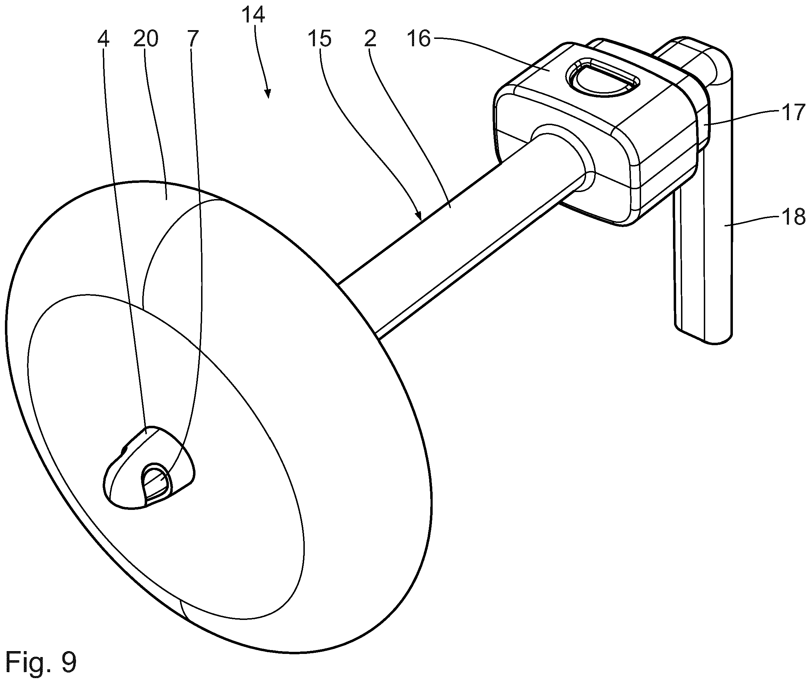

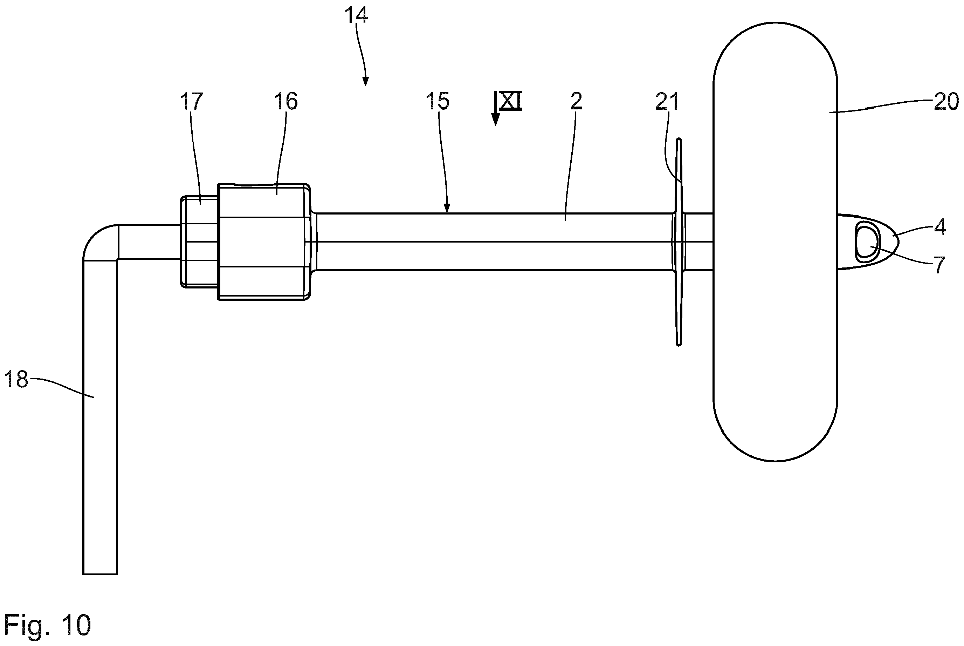

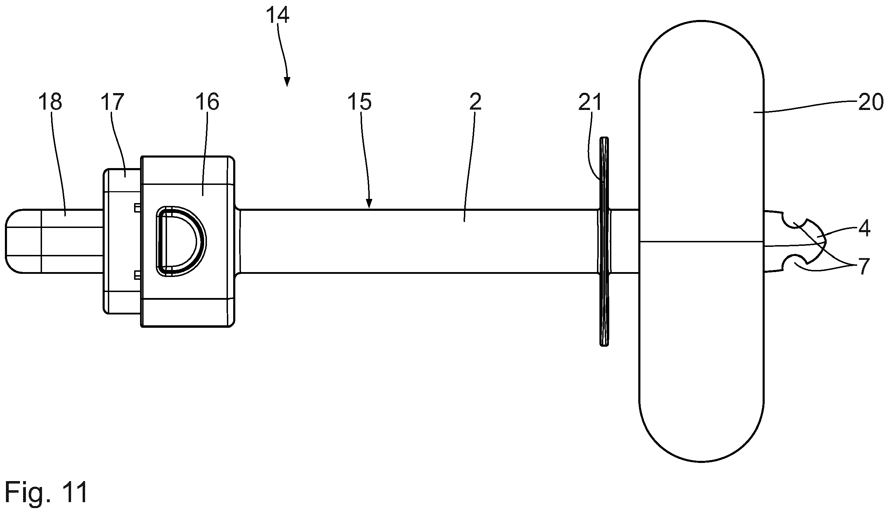

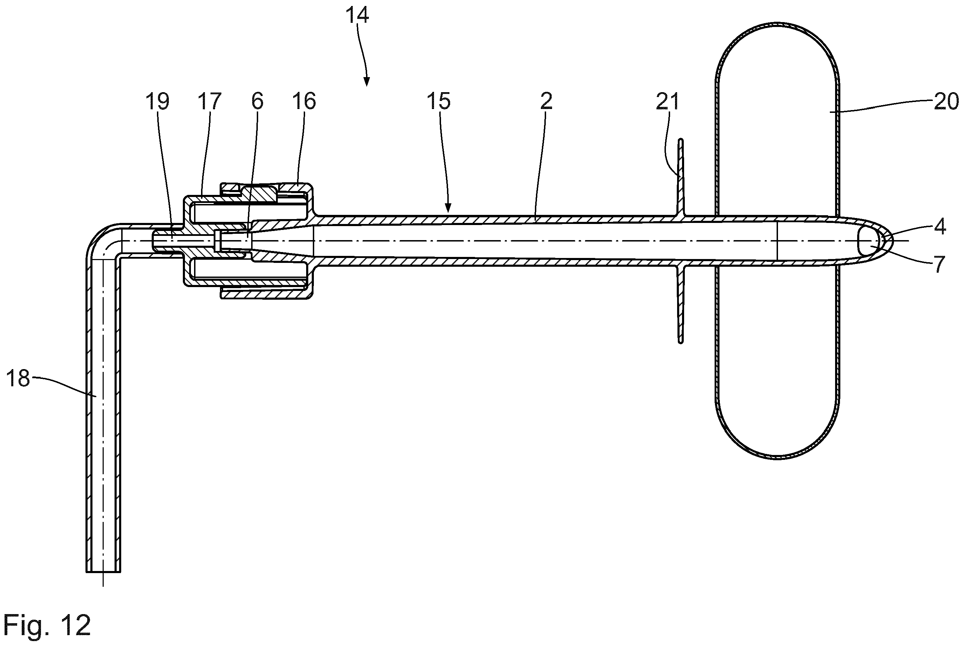

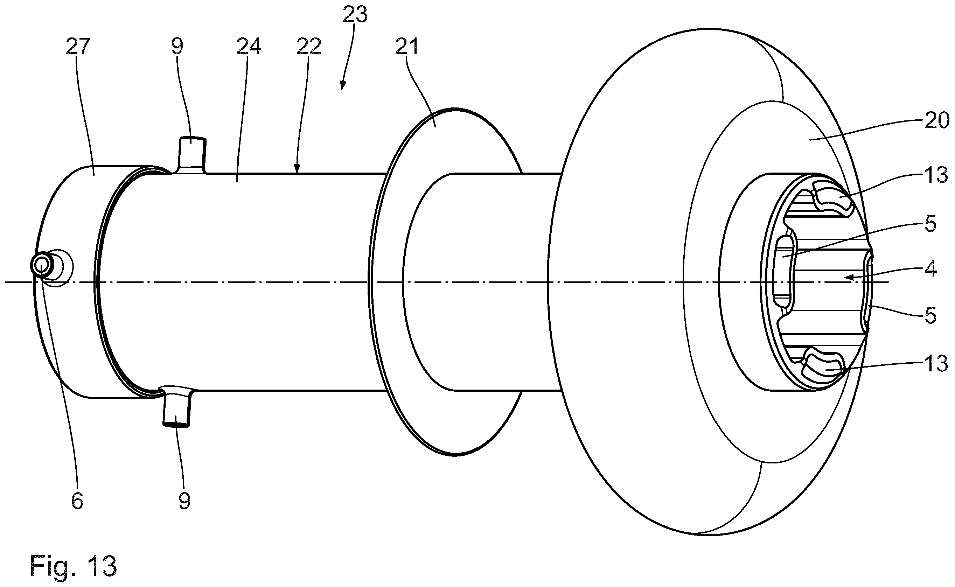

Ein Rektalkatheter-Kunststoffbauteil 1 (vgl. Fig. 6) ist Bestandteil einer Rektalkatheter-Baugruppe, für die Ausführungen nachfolgend noch im Zusammenhang mit den Fig. 9 bis 17 beschrieben werden. Je nach Ausführung der Rektalkatheter-Baugruppe kann diese minuten-, stunden-, tage- oder wochenweise an bzw. in einem Patienten zum Einsatz kommen.

Das Rektalkatheter-Kunststoffbauteil 1 ist als einstückiges Spritzgussbauteil ausgeführt. Das Rektalkatheter-Kunststoffbauteil 1 besteht aus einem Thermoplast-Material. Eine Shore-Härte des Materials kann im Bereich zwischen 80A und 90A liegen. Ein derartiges Material wird auch als semihartes Thermoplast-Material bezeichnet. Das Rektalkatheter-Kunststoffbauteil 1 kann aus Polyurethan gefertigt sein.

Das Kunststoffbauteil 1 hat einen zylindrischen Grundkörper 2 mit einer äußeren Mantelwand. Ein Konnektor-Bauteilende 3 des Kunststoffbauteils 1, welches bei der Ausführung nach Fig. 6 als Anschlussflansch ausgeformt ist, dient zum Anschluss eines Peripherieschlauches (vgl. die Ausführungen nach den Fig. 9 bis 17) zum Zuführen und/oder Abführen von Medien. Die gesamte Rektalkatheter-Baugruppe kann zum Verabreichen eines Einlaufs und/oder zur einmaligen oder fortwährenden Darmreinigung genutzt werden.

Weiterhin hat das Kunststoffbauteil 1 ein freies Bauteilende 4 zum rektalen Einführen durch den Anus in den Darm eines Patienten.

Mindestens ein Medienlumen, beim Ausführungsbeispiel nach Fig. 6 genau ein Medienlumen 5, verläuft längs des Grundkörpers 2 zwischen mindestens einem Medien-Konnektorport 6 zum Anschluss des Peripherieschlauches, ausgeführt am Konnektor-Bauteilende 3, und mindestens einer Medienöffnung, bei der Ausführung nach Fig. 6 vier Medienöffnungen 7, ausgeführt im freien Bauteilende 4. Das freie Bauteilende 4 ist als ballig ausgeführter, in etwa domförmiger, hohler Abschluss des zylindrischen Grundkörpers 2 gestaltet. Die Medienöffnungen 7 sind radial in der Mantelwand des Grundkörpers 2 ausgeführt.

Längs des Grundkörpers 2 verläuft weiterhin mindestens ein weiteres Ballon-Befülllumen, beim Kunststoffbauteil 1 genau ein Ballon-Befülllumen 8. Das Ballon-Befülllumen 8 verläuft zwischen einem Befüll-Konnektorport 9, der ebenfalls am Konnektor-Bauteilende 3 ausgeführt ist, und mindestens einer Ballon-Befüllöffnung, bei dem Kunststoffbauteil 1 nach Fig. 6 genau einer Ballon-Befüllöffnung 10. Letztere ist ausgeführt in einem axialen Ballon-Anschluss-Bereich des Kunststoffbauteil 1 zum Anschluss an einer Ballon-Manschette, die nachfolgend noch im Zusammenhang mit den Ausführungen nach den Fig. 9 bis 17 beschrieben ist. Die Ballon-Befüllöffnung 10 ist radial in der äußeren Mantelwand des zylindrischen Grundkörpers 2 ausgeführt.

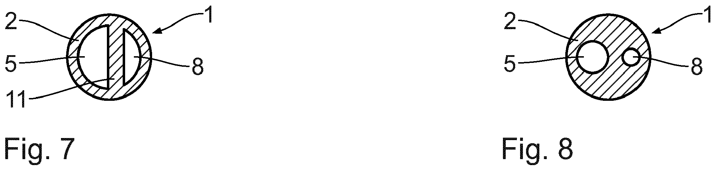

Fig. 7 und 8 zeigen zwei Beispiele für die Querschnittsgestaltung einerseits des Medienlumens 5 und andererseits des Ballon-Befülllumens 8.

Bei der Ausführung nach Fig. 7 sind die beiden Lumen 5, 8 über einen längs des Grundkörpers 2 verlaufenden Steg 11 voneinander getrennt. Der Steg 11 ist einstückig in den Grundkörper 2 eingeformt.

Bei der Ausführung nach Fig. 8 weisen die beiden Lumen 5, 8 jeweils einen konvexen, nämlich kreisförmig runden oder ovalen, Querschnitt auf.

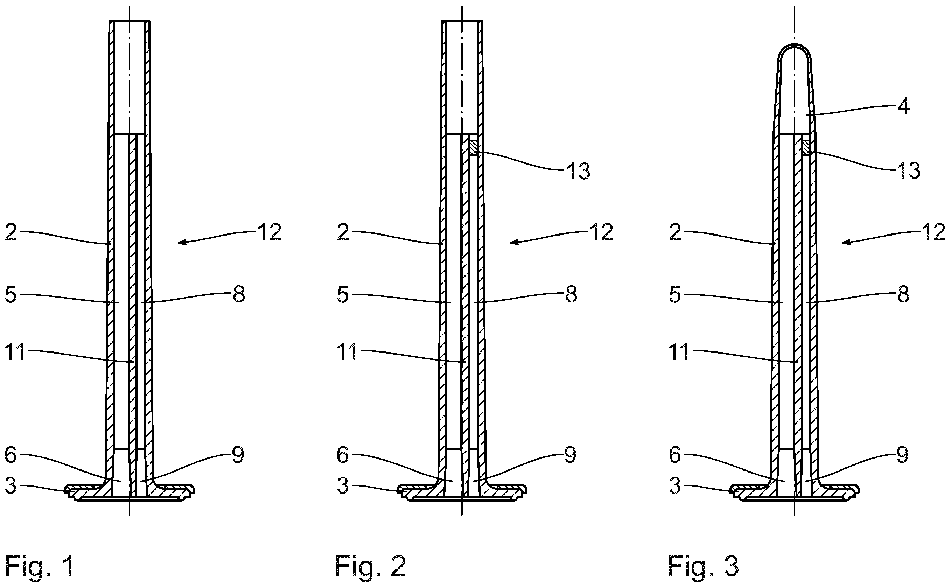

Die Fig. 1 bis 6 zeigen Momentandarstellungen bei der Herstellung des Rektalkatheter-Kunststoffbauteils 1.

Fig. 1 zeigt einen Vorformling, der Spritzguss hergestellt ist und den Grundkörper 2, das Medienlumen 5, das Ballon-Befülllumen 8 sowie das Konnektor-Bauteilende 3 aufweist. Ein freies Ende dieses Vorformlings 12 ist als offenes Rohrende gestaltet, in welches die beiden Lumen 5, 8 ausmünden.

Fig. 2 zeigt das Ergebnis eines nachfolgenden Herstellungsschritts, bei dem ein axiales Ende des Ballon-Befülllumens 8 mit einem Verschlussstopfen 13 verschlossen ist. Bei dem Verschlussstopfen 13 kann es sich um einen Klebstoff-Stopfen handeln.

Fig. 3 zeigt das Resultat eines nachfolgenden Herstellungsschritts, bei dem über einen thermischen Umformprozess und/oder über einen materialabtragenden Prozess aus dem freien Rohrende des Vorformlings 12 das freie Bauteilende 4 geformt ist.

Fig. 4 zeigt das Resultat eines nachfolgenden Herstellungsschritts, bei dem in die äußere Mantelwand des Grundkörpers 2 die Ballon-Befüllöffnung 10 gestanzt ist.

Die Fig. 5 und 6 zeigen die Resultate nachfolgender Schritte zur Herstellung des Kunststoffbauteils 1, bei denen in das freie Bauteilende 4 die Medienöffnungen 7 gestanzt sind.

Anhand der Fig. 9 bis 12 wird nachfolgend eine weitere Ausführung eines Rektalkatheters-Kunststoffbauteils 15 als Bestandteil einer Rektalkatheter-Baugruppe 14 erläutert. Komponenten, die denjenigen, die vorstehend unter Bezugnahme auf die Fig. 1 bis 8 bereits erläutert wurden, tragen die gleichen Bezugsziffern und werden nicht nochmals im Einzelnen diskutiert.

Ein Rektalkatheter-Kunststoffbauteil 15 unterscheidet sich vom Kunststoffbauteil 1 durch die Gestaltung eines Konnektor-Bauteilendes 16. Letzteres ist als Konnektorbuchse gestaltet, in welche ein weiteres, separates Konnektor-Bauteil 17 der Rektalkatheter-Baugruppe 14 einrastet. Die Rektalkatheter-Baugruppe 14 hat also einen zweiteiligen Konnektor zur Verbindung des Rektalkatheter-Kunststoffbauteils 15 mit einem Peripherieschlauch 18 zum Zuführen und/oder Abführen von Medien. Der Peripherieschlauch 18 ist als Mehrlumenschlauch einerseits zum Zuführen von Wasser für die Ballon-Befüllung und andererseits zur Medienzu- bzw. abführung ausgeführt. Der Medien-Konnektorport 6 des Konnektor-Bauteilendes 16 steht über ein Konnektorlumen 19 des separaten Konnektor-Bauteils 17 mit einem Innenlumen des Peripherieschlauchs 18 in Fluidverbindung.

Zur Rektalkatheter-Baugruppe 14 gehört weiterhin eine Ballon-Manschette 20, die im Anschlussbereich des Kunststoffbauteils 15 an diesem fixiert ist. Die Manschette 20 kann dabei mit der äußeren Mantelwand des Grundköpers 2 verklebt und/oder verschweißt sein. Bei der Benutzung der Rektalkatheter-Baugruppe 14 kann die Ballon-Manschette 20 mit körperwarmen Wasser gefüllt sein. Die Ballon-Manschette 20 kann aus Polyurethan gefertigt sein.

Das Rektalkatheter-Kunststoffbauteil 15 hat weiterhin einen Ringanschlag 21, der einstückig an die äußere Mantelwand des Grundkörpers 2 angeformt ist. Der Ringanschlag 21 liegt axial beabstandet zur Ballon-Manschette 20 so, dass letztere zwischen dem Ringanschlag 21 und dem freien Bauteilende 4 angeordnet ist, wo auch der Anschlussbereich des Kunststoffbauteils 15 liegt. Der Ringanschlag 21 wirkt mit der Ballon-Manschette 20 zur Fixierung der Rektalkatheter-Baugruppe 14 im Darm zusammen.

Bei der Benutzung der Rektalkatheter-Baugruppe 14 wird letztere mit zunächst leerer Ballon-Manschette 20 über den Anus in den Darm des Patienten eingeführt, bis der Sphinkter des Darms axial zwischen der Ballon-Manschette 20 und dem Ringanschlag 21 liegt. Anschließend wird über das Ballon-Befülllumen 8 körperwarmes Wasser in die Ballon-Manschette 20 eingefüllt und die Rektalkatheter-Baugruppe 14 somit im Darm fixiert. Es kann nun über das Medienlumen 5 ein Zuführen und/oder Abführen von Medien zwischen der Peripherie und dem Darm erfolgen. Die radiale Anordnung der Medienöffnungen 7 in der Mantelwand des freien Bauteilendes 4 ergibt ein schonendes Zuführen von Medien.

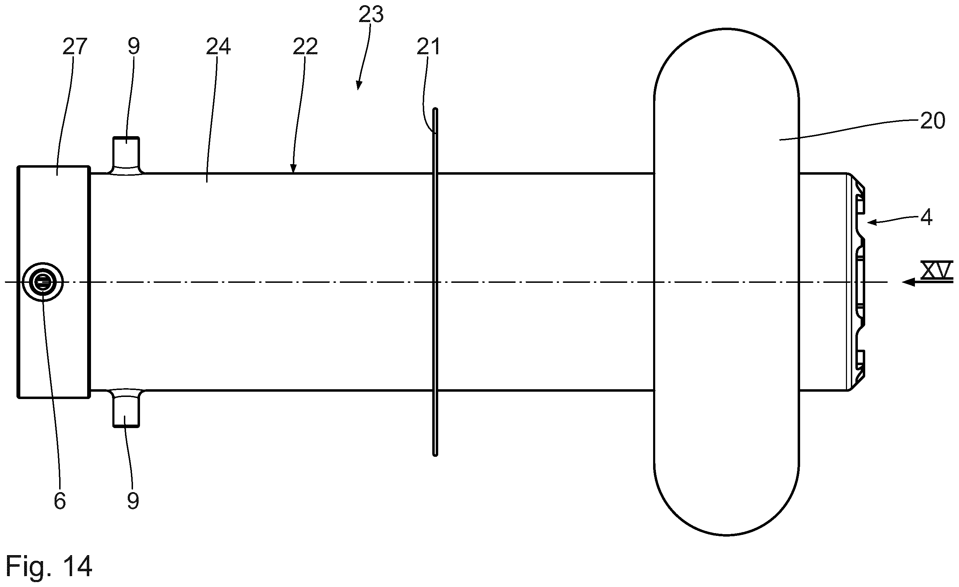

Anhand der Fig. 14 bis 17 wird nachfolgend eine weitere Ausführung eines Rektalkatheter-Kunststoffbauteils 22 für eine weitere Ausführung einer Rektalkatheter-Baugruppe 23 beschrieben. Komponenten, die denjenigen, die vorstehend unter Bezugnahme auf die Fig. 1 bis 12 bereits erläutert wurden, tragen die gleichen Bezugsziffern und werden nicht nochmals im Einzelnen diskutiert.

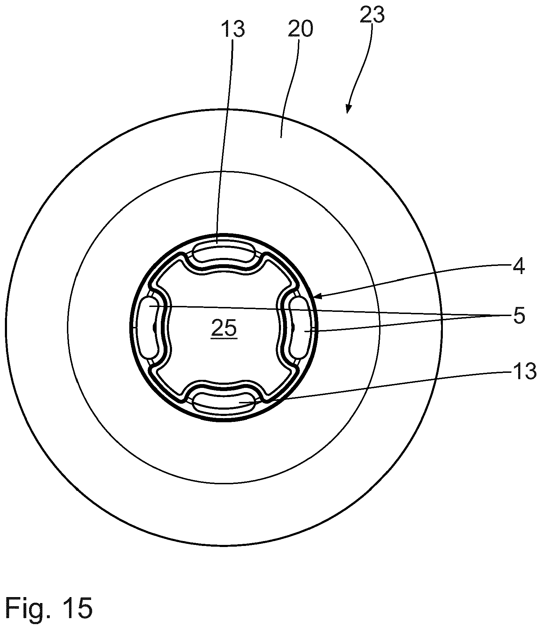

Die Rektal-Baugruppe 23 dient zum länger währenden Einsatz und ermöglicht das Einführen eines zusätzlichen Darmkatheters. Ein Grundkörper 24 des Rektalkatheter-Kunststoffbauteils 22 hat hierzu ein längs des Grundkörpers 24 verlaufendes Katheterlumen 25 (vgl. Fig. 15) zum Einführen des weiteren Darmkatheters. Das Rektalkatheter-Kunststoffbauteil 22 hat vier weitere Lumen, von denen zwei als Medienlumen 5 und zwei als Ballon-Befülllumen 8 genutzt werden können. Die Ballon-Befülllumen 8 weisen an ihren axial freien Enden jeweils einen Verschlussstopfen 13 auf.

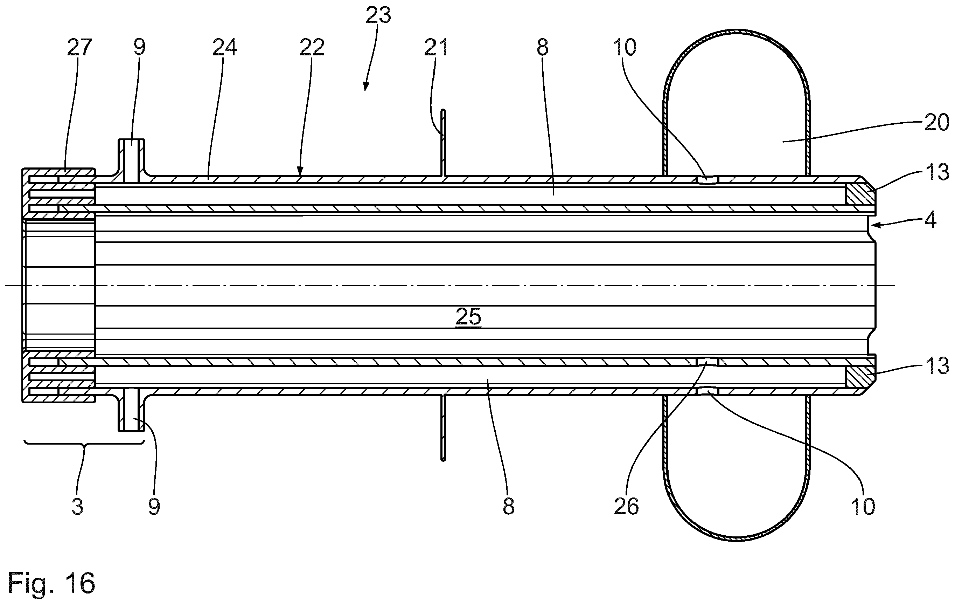

Beim Rektalkatheter-Kunststoffbauteil 22 ist das Konnektor-Bauteilende 3 als axialer Endabschnitt des Grundkörpers 24 ausgestaltet. Wie am Beispiel des in der Fig. 16 unteren Befülllumens 8 dargestellt, kann letzteres über eine Stanzung 26 auch mit dem Katheterlumen 25 in Fluidverbindung stehen. Ein Medien-Konnektorport 6 ist bei der Rektalkatheter-Baugruppe 23 in einer den Grundkörper 24 an dem freien Bauteilende 4 gegenüberliegenden Ende verschließenden Endkappe 27 ausgeführt. Weitere Ports 9, die beispielsweise als Befüll-Konnektorports genutzt werden können, sind direkt an die äußere Mantelwand des Grundkörpers 24 angeformt. Die Endkappe 27 hat eine zentrale Durchtrittsöffnung, die mit dem Katheterlumen 25 des Rektalkatheter-Kunststoffbauteils 22 fluchtet.

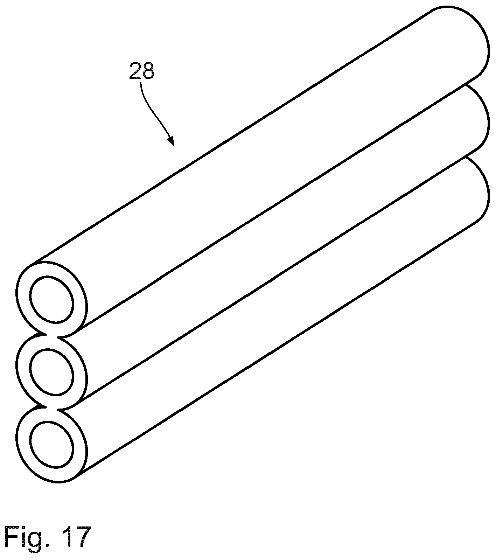

Fig. 17 zeigt eine Ausführung eines Peripherieschlauchs 28 für die Rektalkatheter-Baugruppe 23, die beispielhaft als Dreilumenschlauch ausgeführt ist. Diese drei Lumen können an die Ports 6 sowie 9 der Rektalkatheter-Baugruppe 23 angeschlossen werden.

Bei der Nutzung der Rektalkatheter-Baugruppe 23 kann ein zusätzlicher, nicht dargestellter Darmkatheter durch das Katheterlumen 25 des Rektalkatheter-Kunststoffbauteils 22 in den Darm des Patienten eingeführt werden.

## Patentansprüche

1. Rektalkatheter-Kunststoffbauteil (1; 15; 22) für eine Rektalkatheter-Baugruppe (14; 23),
- mit einem zylindrischen Grundkörper (2; 24) mit einer äußeren Mantelwand,
- mit einem Konnektor-Bauteilende (3; 16) zum Anschluss eines Peripherieschlauches (18; 28) zum Zuführen und/oder Abführen von Medien,
- mit einem freien Bauteilende (4) zum rektalen Einführen,
- mit mindestens einem längs des Grundkörpers (2; 24) verlaufenden Medienlumen (5) zwischen mindestens einem Medien-Konnektorport (6), ausgeführt am Konnektor-Bauteilende (3; 16), und mindestens einer Medienöffnung (7), ausgeführt im freien Bauteilende (4),
- mit mindestens einem weiteren längs des Grundkörpers (2; 24) verlaufenden Ballon-Befülllumen (8) zwischen einem Befüll-Konnektorport (9), ausgeführt am Konnektor-Bauteilende (3; 16), und mindestens einer Ballon-Befüllöffnung (10), ausgeführt in einem Ballon-Anschluss-Bereich des Kunststoffbauteils (1; 15; 22) zum Anschluss einer Ballon-Manschette (20),
- wobei das Rektalkatheter-Kunststoffbauteil (1; 15; 22) einstückig ausgeführt ist, und
- wobei das Rektalkatheter-Kunststoffbauteil (1; 15; 22) aus einem Thermoplast-Material besteht.

2. Rektalkatheter-Kunststoffbauteil nach Anspruch 1, **dadurch gekennzeichnet, dass** benachbarte Lumen (5, 8) über einen längs des Grundkörpers (2; 24) verlaufenden Steg (11) voneinander getrennt sind.

3. Rektalkatheter-Kunststoffbauteil nach Anspruch 1 oder zwei **dadurch gekennzeichnet, dass** die Lumen (5, 8) einen konvexen Querschnitt aufweisen.

4. Rektalkatheter-Kunststoffbauteil nach einem der Ansprüche 1 bis 3, **gekennzeichnet durch** eine Ausführung als Spritzgussbauteil.

5. Rektalkatheter-Kunststoffbauteil nach einem der Ansprüche 1 bis 4, **gekennzeichnet durch** einen einstückig an die Mantelwand des Grundkörpers (2; 24) angeformten Ringanschlag (21).

6. Rektalkatheter-Kunststoffbauteil nach einem der Ansprüche 1 bis 5, **gekennzeichnet durch** ein längs des Grundkörpers (24) verlaufendes Katheterlumen (25) zum Einführen eines weiteren Darmkatheters.

7. Verfahren zum Herstellen eines Rektalkatheter-Kunststoffbauteils (1; 15; 22) nach einem der Ansprüche 1 bis 6 mit folgenden Schritten:
- Spritzgießen eines Vorformlings (12), aufweisend den Grundkörper (2; 24) und mindestens zwei darin verlaufender Lumen (5, 8),
- Verschließen eines axialen Endes mindestens eines der Lumen (8), welches als das Ballon-Befülllumen genutzt wird, mit einem Verschlussstopfen (13),
- Stanzen der mindestens einen Ballon-Befüllöffnung (10) in die äußere Mantelwand des Grundkörpers (2; 24).

8. Verfahren nach Anspruch 7, **gekennzeichnet durch** ein Stanzen der mindestens einen Medienöffnung (7) in das freie Bauteilende (4).

9. Verfahren nach Anspruch 7 oder 8, **gekennzeichnet durch** einen thermischen Umformprozess und/oder über einen materialabtragenden Prozess zur Formung eines freien Bauteilendes (4) aus einem freien Rohrende des Vorformlings (12).

10. Rektalkatheter-Baugruppe
- mit einem Rekatlkatheter-Kunststoffbauteil (1; 15; 22) nach einem der Ansprüche 1 bis 6,
- mit einer Ballon-Manschette (20), die im Ballon-Anschluss-Bereich am Rektalkatheter-Kunststoffbauteil (1; 15; 22) fixiert ist.

11. Rektalkatheter-Baugruppe nach Anspruch 10, **gekennzeichnet durch** einen Peripherieschlauch (18; 28) zum Zuführen und/oder Abführen von Medien.

12. Rektalkatheter-Baugruppe nach Anspruch 10 oder 11, **gekennzeichnet durch** ein separates Konnektor-Bauteil (17) zwischen dem Rektalkatheter-Kunststoffbauteil (15) und dem Peripherieschlauch (18).
